# EUROPEAN PATENT SPECIFICATION

(11) **EP 4 098 191 B1**
(45) Date of publication and mention of the grant of the patent: **04.11.2026**
(21) Application number: 21780040.8
(22) Date of filing: 10.03.2021
(51) Int. Cl.: A61B 5/145, A61B 5/155, A61B 5/151, A61B 5/157

(54) **CONTINUOUS GLUCOSE MONITORING APPARATUS**
GERÄT ZUR KONTINUIERLICHEN GLUKOSEÜBERWACHUNG
APPAREIL DE SURVEILLANCE CONTINUE DU GLUCOSE

(30) Priority: 31.03.2020 KR 20200038783
(43) Date of publication of application: 07.12.2022
(73) Proprietor: i-Sens, Inc., Seoul 06646 (KR)
(72) Inventor: CHAE, Kyung Chul, Seoul 06646 (KR); CHOI, Hyun Ho, Seoul 06646 (KR); RYU, Goang Yel, Seoul 06646 (KR); WANG, Ji Hoon, Seoul 06646 (KR); LEE, Jin Won, Seoul 06646 (KR)
(74) Representative: BCKIP Part mbB
(86) International application number: PCT/KR2021/002978
(87) International publication number: WO 2021/201457

(56) References cited:
- EP-A1- 2 950 101
- EP-A1- 3 329 842
- EP-A1- 3 733 064
- JP-A- 2009 034 425
- JP-A- 2012 205 786
- JP-A- 2012 205 786
- JP-B2- 6 391 469
- KR-A- 20200 014 000
- KR-B1- 101 773 583

## Description

### TECHNICAL FIELD

The present invention relates to a continuous blood glucose measurement apparatus. More specifically, the present invention relates to a continuous blood glucose measurement apparatus in which, by supplying power to a body attachable unit through manipulation of a user, such as separation and removal of a protection cap, in a state in which the body attachable unit is disposed inside an applicator and initiating operation of the body attachable unit, an operation initiation time point of the body attachable unit is made according to the manipulation of the user just before insertion and attachment to the body, thereby initiating the operation of the body attachable unit at an appropriate time point, even though a separate and additional operation for initiating the operation of the body attachable unit is not performed, the operation of the body attachable unit is initiated only by simply removing the protection cap, thereby improving convenience of use, and, by making an operation way of a power supply switch means in various ways associated with manipulation of separation and removal the protection cap, the convenience of production and use can be further improved.

### BACKGROUND

Diabetes is a chronic medical condition that is common in modern people, and in the Republic of Korea, there are 2 million diabetes patients, about 5% of the total population.

Diabetes occurs when the absolute level of the sugar level in blood is high due to the absolute deficiency or relative insufficiency of insulin, produced by the pancreas, caused by various reasons such as obesity, stress, poor eating habits, and inherited hereditary factors and imbalance regarding glucose in the blood.

The blood usually contains a certain concentration of glucose, and tissue cells gain energy from the glucose.

However, when the glucose is increased excessively more than needed, the glucose cannot be properly stored in the liver, muscle, or adipose tissue and is accumulated in the blood, because of this, patients with diabetes maintain a much higher blood glucose level than normal people, and as excessive blood glucose passes through the tissues and is discharged into the urine, it results in deficiency of glucose, which is absolutely necessary for all tissues of the body, thereby causing abnormalities in respective body tissues.

Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown, and further progression of diabetes leads to complications such as visual disturbances, hypertension, kidney disease, paralysis, periodontal disease, muscle spasms and neuralgia, as well as gangrene.

In order to diagnose diabetes beforehand and manage to prevent the progression of diabetes into complications associated therewith, systematic blood glucose measurement and treatment should be performed.

For diabetes patients as well as people having higher than normal blood glucose, even though diabetes has not yet developed, medical device manufacturers offer a variety of blood glucose meters to measure blood glucose levels at home.

Glucose measuring devices may be categorized into a single time measurement type measuring a blood glucose level and collecting blood from a fingertip by a user every single time and a continuous measurement type attaching a glucose monitoring system to the belly or an arm of the user and continuously measuring blood glucose levels.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

Recently, to overcome such a drawback, continuous glucose monitoring systems (CGMSs) inserted into the human body to measure a blood glucose level every few minutes have been developed, and therefore easily perform the management of diabetics and responses to an emergency situation.

Additionally, the blood-collecting glucose monitoring system performs the glucose measurement by collecting blood by pricking a pain-sensitive fingertip with a needle by the diabetes patients themselves, and therefore, the blood collecting process may cause pain and aversion. To minimize such pain and aversion, research and development regarding the CGMSs, which can continuously measure glucose levels by inserting a needle-shaped sensor into a portion of the human body, such as the belly or an arm, which is less pain sensitive, have been undertaken, and furthermore, research and development of non-invasive glucose monitoring systems for measuring glucose without collecting blood have been actively undertaken.

Over the past 40 years, non-invasive glucose monitoring systems have been studied regarding various methods of measuring glucose without collecting blood, for example, optical methods, electrical methods, exhalation measurement methods, and the like. Cygnus Corporation, Redwoo City, Calif., U.S.A, has developed and launched the Glucowatch^{®} G2 Biographer, a wrist watch type, using reverse iontophoresis, but the sales of this product were stopped in 2007, because of many problems, such as skin stimulation issues and qualification approval issues, malfunction caused by sweating, and low reliability in measurement of hypoglycemia comparing with hyperglycemia. Although a variety of non-invasive glucose monitoring techniques have been introduced and reported to date, there have been no practical uses due to low reliability or accuracy.

A continuous glucose monitoring system includes a sensor module inserted and attached to the skin of the human body and measuring a blood glucose level by extracting body fluid, a transmitter transmitting the blood glucose level measured by the sensor module to a terminal, the terminal outputting the received blood glucose level, and any other appropriate component. The sensor module includes a needle-shaped sensor probe for insertion into subcutaneous fat to extract interstitial fluid and any other appropriate component. A separate applicator for attaching the sensor module to the body is used.

Those continuous glucose monitoring systems are manufactured to have a wide variety of types depending on their manufacturers, and are used in a variety of methods. However, the most of the continuous glucose monitoring systems are manufactured and distributed as a type that a one-time use sensor module is attached to the human body using an applicator, the user must perform several steps for the operation of the applicator for attaching a sensor module for single use to the body, and after attaching the sensor module to the body, various subsequent procedures, such as pulling out the needle by the user himself or herself, need to be performed.

For example, a packaging of the sensor module for single use must be removed and the sensor module needs to be accurately inserted into the applicator, and in a state in which the sensor module is inserted to the applicator, the sensor module is inserted into the skin by manipulating the applicator, after the insertion, a manipulation, such as pulling the needle of the sensor module from the skin by the user himself or herself, is required to be performed, and a manipulation, such as coupling a separate transmitter to the sensor module for transmitting the blood glucose measurement result to the user terminal, has to be performed.

Accordingly, there is a problem in that the manipulation for measuring blood glucose using the continuous blood glucose measurement apparatus is very cumbersome and inconvenient. In addition, because a manipulation for an operation initiation of the sensor module and the transmitter is not performed by the user, the accuracy of the blood glucose measurement result is lowered and the lifespan of the apparatus is reduced.

EP 3 329 842 A1 discloses a device to monitor an analyte in body fluid. Examples of this document disclose continuous or discrete acquisition of analyte related data from a transcutaneously positioned analyte sensor automatically or on demand upon request from a user.

JP 2012-205786 A addresses the problem of how to reduce power consumption during measurement while retaining the operability of a subject in a blood sugar measuring device. To this end, a blood sugar measuring device is disclosed. The device includes a detection part which executes a detection process for detecting the intensity of reflection light by irradiating a blood sample with light and computes a blood sugar value based on the result of detection by the detection part. The blood sugar measuring device further includes: a chip detachably attached to the detection part, a protection cap detachably attached to the detection part to protect the detection part, an ejector which can remove the chip and the protection cap, a sensor for detecting the operation of the ejector, and a control part for controlling the motion of the blood sugar measuring device according to the result of detection by the sensor.

EP 2 950 101 A1 discloses a blood glucose meter. A cap that protects an optical measuring unit of the blood glucose meter is provided, and a micro switch that detects a state in which the cap is attached to or removed from the housing is provided. When an input/output control unit detects that the cap is removed from the housing, the input/output control unit moves from a power saving mode to a measuring mode.

### SUMMARY OF THE INVENTION

### Technical Problem

The present invention is invented to solve problems in conventional technique, and the purpose of the present invention is for providing a continuous blood glucose measurement apparatus in which, by supplying power to a body attachable unit through manipulation of a user, such as separation and removal of a protection cap, in a state in which the body attachable unit is disposed inside an applicator and initiating operation of the body attachable unit, an operation initiation time point of the body attachable unit is made according to the manipulation of the user just before insertion and attachment to the body, thereby initiating the operation of the body attachable unit at an appropriate time point, even though a separate and additional operation for initiating the operation of the body attachable unit is not performed, the operation of the body attachable unit is initiated only by simply removing the protection cap, thereby improving convenience of use.

Another purpose of the present invention is for providing a continuous blood glucose measurement apparatus in which, by making an operation way of a power supply switch means in various ways associated with manipulation of separation and removal the protection cap, the convenience of production and use can be further improved.

### Solution to Problem

To this end, the present invention provides a continuous glucose measurement apparatus in accordance with claim 1, and a continuous glucose measurement apparatus in accordance with claim 5.

The present invention provides a continuous glucose measurement apparatus, comprising: a body attachable unit configured to be insertedly attachable to a body to periodically measure blood glucose; and an applicator in which the body attachable unit is coupled, the applicator configured to outwardly discharge the body attachable unit according to manipulation of a user so that the body attachable unit is insertedly attached to the body, wherein a battery for power supply is mounted inside the body attachable unit, and the body attachable unit is configured to initiate operation by power of the battery supplied according to the manipulation of the user in a state that the body attachable unit is arranged inside the applicator.

According to the invention, a protection cap is separatably coupled to the applicator to block exposure of the body attachable unit to an outside of the applicator, and the applicator further comprises a power supply switch means to supply the power of the battery to the body attachable unit in association with manipulation of separation and removal of the protection cap.

According to the invention, the body attachable unit comprises a housing of which a bottom surface is attachable to skin, and a PCB board arranged inside the housing to be electrically connected to the battery. The body attachable unit further includes a sensor module arranged inside the housing such that an end of the sensor module outwardly protrudes from the bottom surface of the housing, and electrically connected to the PCB board. The power supply switch means is configured to supply the power from the battery to the PCB board according to the separation and removal of the protection cap.

According to one aspect of the invention, the power supply switch means comprises: a board contact point formed at the PCB board; a battery contact point connected to the battery; and a switch terminal mounted movably inside the housing to be capable of contacting both the board contact point and the battery contact point simultaneously, the switch terminal moves in association with the manipulation of the separation and removal of the protection cap such that the switch terminal contacts both the board contact point and the battery contact point simultaneously to electrically connect between the board contact point and the battery contact point.

Additionally, the power supply switch means may comprise: an elastic member configured to apply an elastic force to the switch terminal in a direction in which the switch terminal contacts the board contact point and the battery contact point simultaneous; and a manipulation protrusion formed to protrude at one side of the protection cap, the manipulation protrusion configured to press the switch terminal so that the switch terminal is spaced apart from the board contact point and the battery contact point.

Further, an insertion guide portion protruded toward an inward direction so that insertion of the manipulation protrusion of the protection cap is guided may be formed at the bottom surface of the housing.

Additionally, a blocking film of a soft material configured to be deformable by pressure of the manipulation protrusion and block an inner space of the housing from an outside of the housing may be arranged at an end portion of protrusion of the insertion guide portion.

According to another aspect of the invention, the power supply switch means comprises: a switch element configured to electrically connect or disconnect between the battery and the PCB board according to an electrical signal inputted to an enable terminal; a switch contact point having one end grounded and an other end connected to the enable terminal; and a connection terminal formed at one side of the protection cap and configured to electrically connect or disconnect between the one end and the other end of the switch contact point according to whether the protection cap is separated and removed from the applicator or not, wherein, when the connection terminal electrically connects between the one end and the other end of the switch contact point, a low electric signal is inputted to the enable terminal, and when the connection terminal electrically disconnects between the one end and the other end of the switch contact point, a high electric signal is inputted to the enable terminal by the power of the battery.

In addition, the switch contact point may be formed to be positioned in an inside space of the housing, a manipulation protrusion protruding toward the switch contact point is formed at one side of the protection cap, the connection terminal may be formed at an end portion of the manipulation protrusion, and an insertion guide portion protruding in an inward direction so that insertion of the manipulation protrusion of the protection cap is guided may be formed at the bottom surface of the housing.

### Advantageous Effects of Invention

According to the present invention, by supplying power to a body attachable unit through manipulation of a user, such as separation and removal of a protection cap, in a state in which the body attachable unit is disposed inside an applicator and initiating operation of the body attachable unit, an operation initiation time point of the body attachable unit is made according to the manipulation of the user just before insertion and attachment to the body, thereby initiating the operation of the body attachable unit at an appropriate time point, even though a separate and additional operation for initiating the operation of the body attachable unit is not performed, the operation of the body attachable unit is initiated only by simply removing the protection cap, thereby improving convenience of use.

Additionally, by making an operation way of a power supply switch means in various ways associated with manipulation of separation and removal the protection cap, the convenience of production and use can be further improved.

### BRIEF DESCRIPTION OF THE DRAWINGS

FIG. 1 is a diagram for schematically illustrating a basic system of a continuous blood glucose measurement apparatus according to an embodiment of the present invention.
FIG. 2 is a diagram for schematically illustrating a structure of an applicator of a continuous blood glucose measurement apparatus according to an embodiment of the present invention.
FIG. 3 is a diagram for schematically illustrating a body attachable unit of a continuous blood glucose measurement apparatus according to an embodiment of the present invention.
FIG. 4 is a diagram for explaining a body insertion process of a body attachable unit through an applicator according to an embodiment of the present invention.
FIGS. 5 and 6 are diagrams for illustrating a coupling structure between a body attachable unit and a protection cap according to an embodiment of the present invention.
FIG. 7 is a conceptual view for conceptually illustrating a configuration of a power supply switch means according to a first embodiment of the present invention.
FIG. 8 is a diagram for schematically illustrating a configuration for implementing the power supply switch means shown in FIG. 7.
FIG. 9 is a conceptual view for conceptually illustrating a configuration of a power supply switch means according to a second embodiment of the present invention.
FIG. 10 is a diagram for schematically illustrating a configuration for implementing the power supply switch means shown in FIG. 9.

### DETAILED DESCRIPTION OF EMBODIMENTS

Hereinafter, embodiments of the present invention will be described in detail with reference to the accompanying drawings. Throughout this document, reference should be made to the drawings, in which the same reference numerals and symbols will be used to designate the same or like components. Additionally, in the following description of the present invention, detailed descriptions of known functions and components incorporated herein will be omitted in the case that the subject matter of the present invention may be rendered unclear thereby.

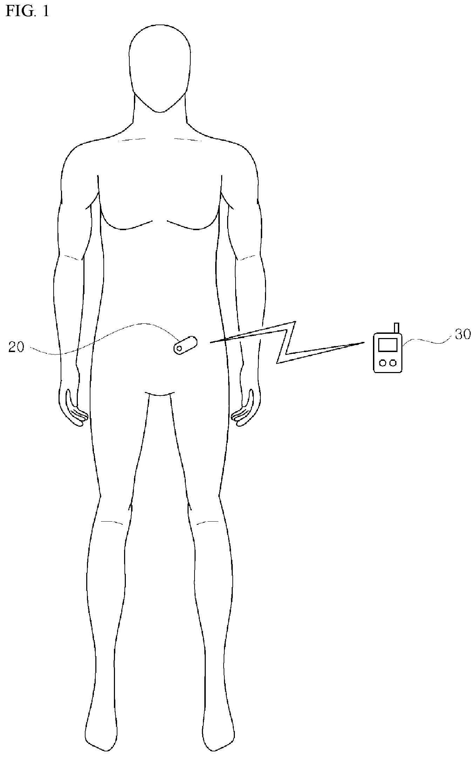

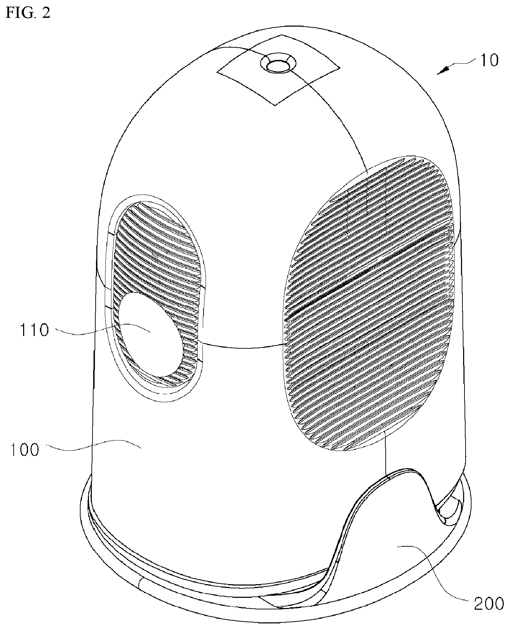

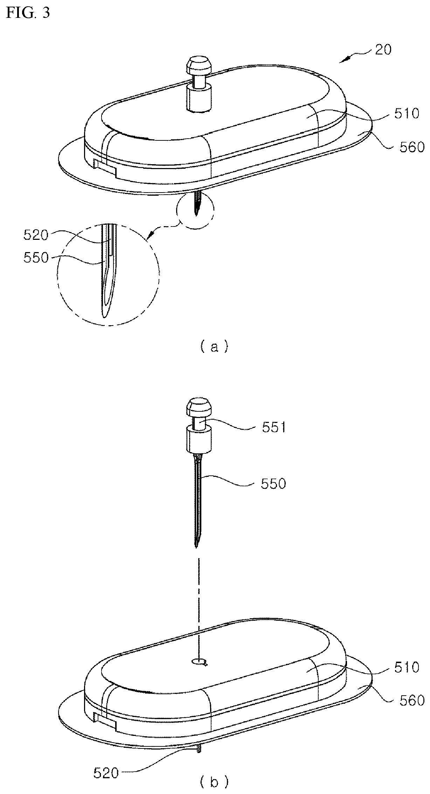

FIG. 1 is a diagram for schematically illustrating a basic system of a continuous blood glucose measurement apparatus according to an embodiment of the present invention, FIG. 2 is a diagram for schematically illustrating a structure of an applicator of a continuous blood glucose measurement apparatus according to an embodiment of the present invention, and FIG. 3 is a diagram for schematically illustrating a body attachable unit of a continuous blood glucose measurement apparatus according to an embodiment of the present invention.

A continuous blood glucose measurement apparatus according to an embodiment of the present invention is configured to attach a body attachable unit (20) having a sensor module (520) insertable into a body using an applicator (10) for continuous blood glucose measurement, to insert and attach the body attachable unit (20) to the body by operating or manipulating the applicator (10) to periodically and continuously measure blood glucose from the body, and to transmit blood sugar measurement information periodically measured through the body attachable unit (20) to a separate terminal (30) to output it.

The body attachable unit (20) can be manufactured as a single unit product by being assembled inside the applicator (10), and, in this case, has a structure of which use method is very simple in a shape in which additional work of a user can be minimized when using the continuous blood glucose measurement apparatus. Of course, various manufacturing ways including an example that the body attachable unit (20) is supplied to the user separately from the applicator (10) and the user inserts the body attachable unit (20) into the inside of the applicator (10) to operate it can be implemented.

The body attachable unit (20) may be configured to be attachable to a human body to periodically measure blood sugar level or glucose by extracting body fluid, and transmit the blood glucose measurement result to an external device such as an external terminal (30) and so on. A sensor module (520) of which one end portion can be inserted into the human body and a wireless communication chip (not shown) configured to wirelessly communicate with the external terminal (30) can be disposed inside the body attachable unit (20).

The applicator (10) is formed such that the body attachable unit (20) is fixedly coupled to the inside of the applicator (10), and the applicator (10) is configured to outwardly discharge the body attachable unit (20) according to the user's pressurizing manipulation to a press button (110).

In this embodiment, the body attachable unit (20) is assembled and produced in a state that the body attachable unit (20) is inserted into the inside of the applicator (10), and is configured to move in an outward discharge direction pursuant to the operation of the applicator (10) by the manipulation of the user and be attached to the human body.

Therefore, a sensor applicator assembly according to an embodiment of the present invention is assembled and manufactured in a state that the body attachable unit (20) is inserted in the inside of the applicator (10) at the manufacturing stage and the body attachable unit (20) can be attached to a skin by only the operation of the applicator (10), and because the sensor applicator assembly (1) is supplied to the user in this state, the user can easily attach the body attachable unit (20) to the skin by only the manipulation simply activating the applicator (10) without extra additional operation for attaching the body attachable unit (20) to the skin. Specifically, since the body attachable unit (20) has the wireless communication chip, no connection with an extra transmitter is needed and therefore it can be used more conveniently.

A separate and additional protection cap (200) can be separably coupled to the applicator (10) in order to block external exposure in a state that the applicator (10) is inserted in the inside of the applicator (10), and it may be configured that the user can attach the body attachable unit (20) to the human body by manipulating the applicator (10) after the protection cap (200) is separated and outwardly discharging the body attachable unit (20) toward a position where the protection cap (200) is removed.

In the embodiment of the present invention, an adhesive tape (560) is provided at a side of the body attachable unit (20) contacting the human body to be attached to the body, to protect the adhesive tape (560) a release paper (not shown) is attached to a surface of the adhesive tape (560) contacting the human body, and the release paper of the adhesive tape (560) may be configured to be separated and removed from the adhesive tape (560) during the operation of separating the protection cap (200) from the applicator (10).

In a state that the body attachable unit (20) is inserted in the inside, the applicator (10) fixes the body the attachable unit (20), and in a state that the body attachable unit (20) is outwardly discharged and moved, the applicator (10) is configured to release the fixed state of the body attachable unit (20). Accordingly, in a state that the body attachable unit (20) is assembled to be inserted in the inside of the applicator (10), the body attachable unit (20) maintains the fixed state, and when the body attachable unit (20) is externally discharged and attached to the skin by actuating the applicator (10), the state fixed between the applicator (10) and the body attachable unit (20) is released, and therefore if the applicator (10) is separated in this state the applicator (10) is separated from the body attachable unit (20) and only the body attachable unit (20) remains on the skin.

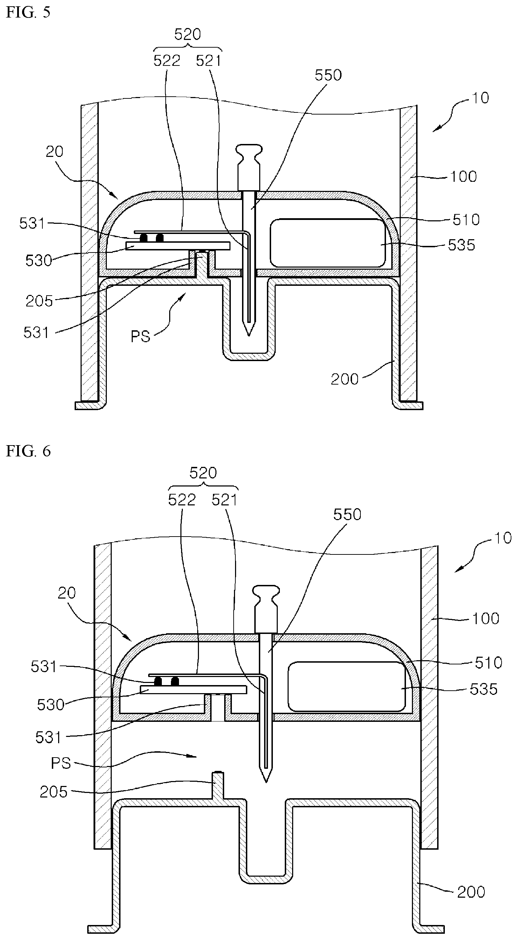

In the body attachable unit (20), the sensor module (520) is disposed in a separate housing (510), and one end portion of the sensor module (520) outwardly protrudes from the bottom surface of the housing (510) so that it can be inserted and attached to the human body. The sensor module (520) may comprise a sensor probe module (521) (See FIG. 5) to be inserted into the human body, and a sensor body module (522) (See FIG. 5) disposed inside the housing (510), and the sensor probe module and the sensor body module are formed as one end portion and another end portion of the sensor unit (520), respectively, and in a bent shape.

In this embodiment, to smoothly perform the body insertion process of the sensor module (520), a separate guide needle (550) may be separatably coupled to the housing (510). The guide needle (550) may surround one end portion of the sensor module (520) and be configured to be inserted together with the sensor module (520) so that one end portion of the sensor module (520) can be stably inserted into the human body.

As shown in FIG. 2, the guide needle (550) may be separatably coupled to the housing (510) in a direction penetrating the top and bottom of the housing (510) of the body attachable unit (20), the guide needle (550) may be formed to have a structure covering the outside of the sensor module (520), and a need head (551) is formed at the upper end portion of the guide needle (550). If the body attachable unit (20) is moved in the direction outwardly discharged by the applicator (10), the guide needle (550) is inserted into the human body first before the sensor module (520) is inserted into the human body and the guide needle (550) may guide the sensor module (520) such that the sensor module (520) can be stably inserted in the skin. The guide needle (550) may be coupled with a needle extracting means (not shown) of the applicator (10) through the needle head (551), and after the body attachable unit (20) is inserted and attached to the human body by the operation of the applicator (10), the guide needle (550) may be configured to be withdrew and removed from the human body by the needle extracting means of the applicator (10).

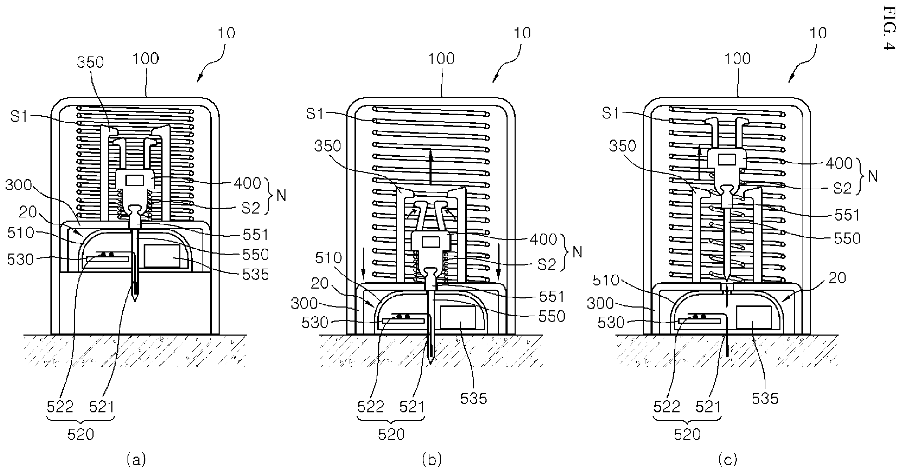

FIG. 4 is a diagram for explaining a body insertion process of a body attachable unit through an applicator according to an embodiment of the present invention, and FIGS. 5 and 6 are diagrams for illustrating a coupling structure between a body attachable unit and a protection cap according to an embodiment of the present invention.

The applicator (10) according to an embodiment of the present invention is a device that operates to insertly attach the body attachable unit (20) to the body by the user's manipulation as described above, and can be configured to comprise a main case (100) with one open side, a plunger body (300) configured to move in a direction of being outwardly discharged from the inside of the main case (100) toward the open side, and a plunger elastic spring (S1) applying an elastic force to the plunger body (300) in a direction in which the plunger body (300) is outwardly discharged, and the body attachable unit (20) is coupled to the plunger body (300) and can be configured to move in a direction of being outwardly discharged together with the plunger body (300).

A manipulation or operation part, specifically, a pressing button (not shown) that a user can manipulate is provided on the outside of the main case (100), the plunger body (300) is coupled and fixed to a first position inside the main case (100), the coupling and fixing are released from the first position according to the manipulation of the manipulation or operation part, and the plunger body (300) is linearly moved to a second position, which is in an outward discharge direction, by the elastic force of the plunger elastic spring (S1). The body attachable unit (20), which is coupled to one end of the plunger body (300), linearly moves in the outward discharge direction and is inserted and attached to the body skin (E) together with the plunger body (300).

The body attachable unit (20) includes a housing (510), a sensor module (520) including a sensor body portion (522) and a sensor probe portion (521), and a guide needle (550) arranged to surround an outside of the sensor probe portion (521) and separatably coupled to the housing (510). A PCB board (530) and a battery (535) configured to supply power to the PCB board (530) are mounted inside the housing (510). A wireless communication chip (not shown) for communication with an external terminal is mounted to the PCB board (530), and the sensor module (520) is electrically connected to the PCB board (530) through an electric contact point (531).

When the body attachable unit (20) is outwardly discharged, the sensor probe portion (521) of the sensor module (520) is arranged to outwardly protrude from the bottom surface of the housing (510) so that the sensor probe portion (521) is inserted into the body skin (E), and the guide needle (550) surrounds an outside of the sensor probe portion (521) and is inserted into the body skin (E) together with the sensor probe portion (521).

After the guide needle (550) is inserted into the body skin (E), the applicator (10) comprises a needle extracting means (N) withdrawing and removing the guide needle (550) from the body skin (E) in a state that the guide needle (550) is inserted to the body skin (E).

The needle extracting means (N) is engaged with the plunger body (300) and can be configured to comprise a needle extracting body (400) coupled with a needle head (551) formed at an upper portion of the guide needle (550), and a needle extracting elastic spring (S2) applying an elastic force to the needle extracting body (400) in a direction opposite to the outward discharge direction. A hook engaging portion (350) capable of constraining the elastic movement of the needle extracting body (400) is formed at the plunger body (300), and an elastic hook (410) engaging with the hook engaging portion (350) is formed at the upper portion of the needle extracting body (400). The elastic hook (410) is engaged with the hook engaging portion (350) so that the needle extracting body (400) is integrally moved together with the plunger body (300) in the outward discharge direction, when it moves by a preset distance, the engagement state between the elastic hook (410) and the hook engaging portion (350) is released by a separate catch release means (not shown), and in this state, the needle extracting body (400) is moved in a direction opposite to the outward discharge direction by the needle extracting elastic spring (S2).

That is, after the applicator (10) is attached to the skin in a state in which a protection cap (200) is separated and removed from the applicator (10) as illustrated in FIG. 4(a), if the press button (110) is pressed, the body attachable unit (20) together with the plunger body (300) is outwardly discharged and the end of the sensor module (520) is inserted into the body skin as shown in FIG. 4(b). At the same time, as shown in FIG. 4(c), the guide needle (550) is withdrawn from the skin by the needle extracting means (N). Thereafter, when the applicator (10) is separated and removed from the skin, only the body attachable unit (20) remains attached to the skin, and the body attachable unit (20) measures blood glucose periodically in this state.

In a continuous blood glucose measurement apparatus according to an embodiment of the present invention, the protective cap (200) is coupled in a state in which the body attachable unit (20) is disposed inside the applicator (10) as described above, and after the protective cap (200) is removed, the body attachable unit (20) can be outwardly discharged to be inserted and attached to the body by the manipulation of pressing the press button of the applicator (10).

At this time, in a state that the protection cap (200) is coupled to the applicator (10), the applicator (10) is configured to prevent the manipulation for outwardly discharging the body attachable unit (20), specifically, the manipulation of pressing the press button. For example, a separate locking protrusion portion may be formed on the protection cap (200) to restrict the pressing movement of the press button in a state in which the protection cap (200) is coupled to the applicator (10).

On the other hand, the continuous blood glucose measurement apparatus according to an embodiment of the present invention is configured to initiate its operation by being supplied from the power of the battery (535) according to the user's manipulation in a state in which the body attachable unit (20) is arranged inside the applicator (10). That is, before the body attachable unit (20) is externally discharged from the applicator (10), the power of the battery (535) may be supplied to the body attachable unit (20) by the user's manipulation.

To this end, a power supply switch means (PS) is provided to perform an operation that the power of the battery (535) is supplied to the body attachable unit (20) in association with the separation and removal operation of the protection cap (200).

The body attachable unit (20) includes a housing (510), a PCB substrate or board (530), and a sensor module (520) as described above, and the battery (535) electrically connectable to the PCB board (530) is mounted inside the housing (510), and at this time, the power supply switch means (PS) operates to supply power from the battery (535) to the PCB board (530) as the protection cap (200) is separated and removed.

As illustrated in FIG. 5 and 6, a manipulation protrusion (205) is formed on one side of the protection cap (200), an insertion guide portion (513) is formed at the housing (510) of the body attachable unit (20) such that the insertion of the manipulation protrusion (205) can be guided, and by using an electric element operating through such a mechanical configuration, when the protection cap (200) is separated and removed as illustrated in FIG. 6, the power supply switch means (PS) is configured to operate to supply power to the PCB board (530).

In this way, in a state in which the body attachable unit (20) is positioned inside the applicator (10), the power is supplied to the body attachable unit (20) to initiate operation through the user's manipulation such as separation and removal of the protection cap (200), and therefore an operation initiation time point of the body attachable unit (20) is made by the user's manipulation immediately before the insertion and attachment to the body, so that the operation of the body attachable unit (20) can be initiated at an appropriate time. In particular, even if a separate additional operation for starting the operation of the body attachable unit (20) is not performed, the operation of the body attachable unit (20) can be initiated by simply removing the protection cap (200), thereby further improving use convenience.

Next, more details regarding the power supply switch means (PS) according to an embodiment of the present invention will be followed.

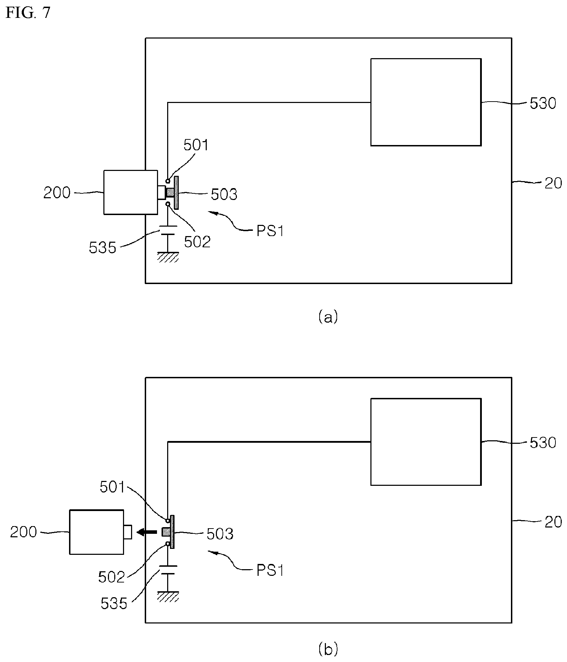

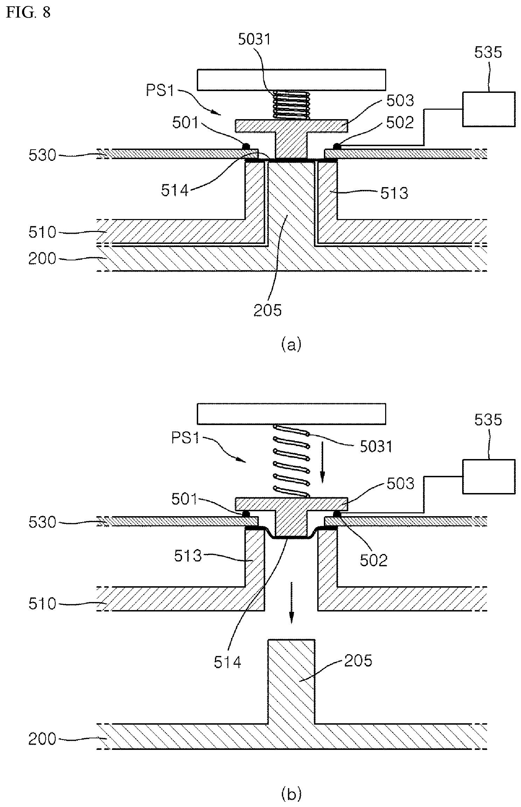

FIG. 7 is a conceptual view for conceptually illustrating a configuration of a power supply switch means according to a first embodiment of the present invention, and FIG. 8 is a diagram for schematically illustrating a configuration for implementing the power supply switch means shown in FIG. 7.

The power supply switch means (PS1) according to the first alternative of the present invention comprises a board contact point (501) formed on the PCB board (530), a battery contact point (502) connected to the battery (535), and a switch terminal (503) mounted movably inside the housing (510) to be capable of simultaneously contacting the board contact point (501) and the battery contact point (502), and the switch terminal (503) is configured to be movable to simultaneously contact the board contact point (501) and the battery contact point (502) in association with the separation removal operation of the protection cap (200) to be electrically connected to each other.

At this time, an elastic member (5031) for applying an elastic force to the switch terminal (503) in a direction in which the switch terminal (503) simultaneously contacts the board contact point (501) and the battery contact point (502) is arranged inside the housing (510). Also, in a state in which the protection cap (200) is coupled to the applicator (10), a manipulation protrusion (205) pressurizing the switch terminal (503) such that the switch terminal (503) can be spaced apart from the board contact point (501) and the battery contact point (502) may be formed to protrude from one surface of the protection cap (200).

In the state in which the protection cap (200) is coupled to the applicator (10) as shown in FIGS. 7(a) and 8(a), the manipulation protrusion (205) presses the switch terminal (503) such that the switch terminal (503) is spaced apart from the board contact point (501) and the battery contact point (502), and accordingly the board contact point (501) and the battery contact point (502) are not connected to each other, so battery power is not supplied to the PCB board (530).

After that, when the user intends to insert and attach the body attachable unit (20) to the body, the protection cap (200) needs to be separated and removed from the applicator (10), and if the protection cap (200) is removed as shown in FIGS. 7 (b) and 8 (b), the manipulation protrusion (205) releases the press applied to the switch terminal (503), and therefore the switch terminal (503) is moved by the elastic force of the elastic member (5031) to simultaneously contact the board contact point (501) and the battery contact point (502). Accordingly, the board contact point (501) and the battery contact point (502) are electrically connected to each other by the switch terminal (503), so that the battery power can be supplied to the PCB board (530) and the body attachable unit (20) starts to operate.

At this time, the insertion guide portion (513) protruding inwardly so that the insertion of the manipulation protrusion (205) of the protection cap (200) can be guided may be formed at the bottom surface of the housing (510). In addition, a blocking film (514) made of a flexible material may be mounted at a protrusion end of the insertion guide portion (513) to block the inner space of the housing (510) from the outside. The blocking film (514) is for performing a waterproof function for the inner space of the housing (510), and may be formed of a soft or malleable material that can be deformable by pressure of the manipulation protrusion (205).

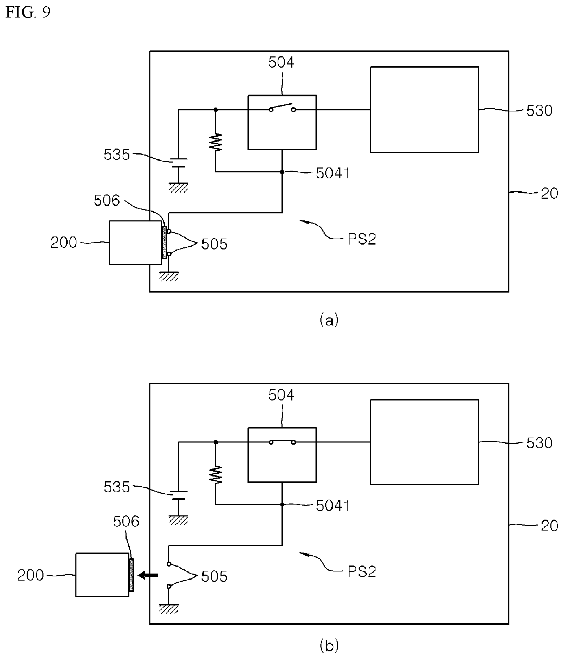

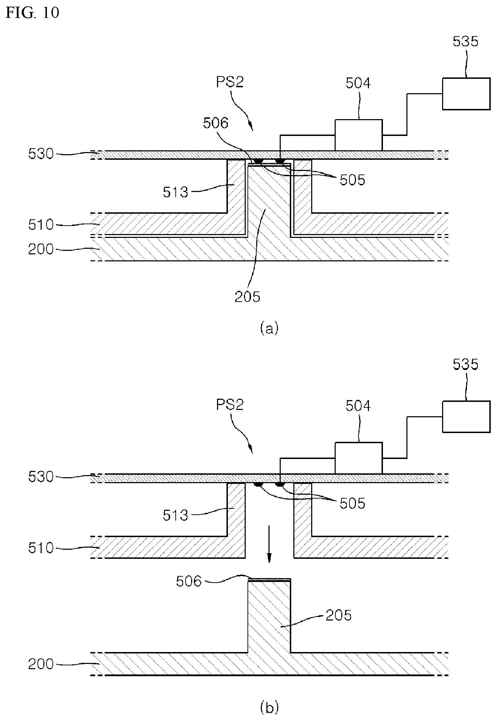

FIG. 9 is a conceptual view for conceptually illustrating a configuration of a power supply switch means according to a second alternative of the present invention, and FIG. 10 is a diagram for schematically illustrating a configuration for implementing the power supply switch means shown in FIG. 9.

A power supply switch means (PS2) according to the second alternative of the present invention is configured to include a switch element (504) configured to electrically connect the battery (535) and the PCB board (530) or block their connection according to an electrical signal input to an enable terminal (5041), a switch contact point (505) having one end grounded and the other end connected to the enable terminal (5041), and a connection terminal (506) formed at one side of the protection cap (200) and configured to electrically connect one end and the other end of the switch contact point (505) or block their connection according to whether the protection cap (200) is separated and removed or not. At this time, when the connection terminal (506) electrically connects between one end and the other end of the switch contact point (505), a low electrical signal is input to the enable terminal (5041), and when the connection terminal (506) blocks the electrical connection between one end and the other end of the switch contact point (505), a high electrical signal is input to the enable terminal (5041) by the power of the battery (535). In addition, the switch contact point (505) may be formed to be positioned in the inner space of the housing (510), and the connection terminal (506) may be formed at an end of the manipulation protrusion (205) of the protection cap (200).

In a state in which the protection cap (200) is coupled to the applicator (10) as shown in FIGS. 9(a) and 10(a), the connection terminal (506) of the manipulation (505) is connected to the switch contact point (505) to electrically connect one end and the other end of the switch contact point (505), and if the protection cap (200) is separated and removed from the applicator (10) as shown in FIGS. 9(b) and 10(b), the connection terminal (506) of the manipulation protrusion (205) is spaced apart from the switch contact point (505) to block the electrical connection between one end and the other end of the switch contact point (505).

When one end and the other end of the switch contact point (505) are electrically connected by the connection terminal (506), a low electrical signal is input to the enable terminal (5041) of the switch element (504), and in this case, the switch element (504) operates to electrically disconnect the battery (535) and the PCB board (530). Accordingly, the power of the battery (535) is not supplied to the PCB board (530).

When the connection terminal (506) is spaced apart from one end and the other end of the switch contact point (505), one end and the other end of the switch contact point (505) are electrically disconnected, and a high electrical signal is inputted to the enable terminal (5041) of the switch element (504). In this case, the switch element (504) operates to electrically connect the battery (535) and the PCB board (530), and the power of the battery (535) is supplied to the PCB board (530) to initiate the operation of the body attachable unit (20).

These operation methods of the switch contact point (505) and the switch element (504) are exemplary, and various other methods may be applied and a TR (transistor), FET (field effect transistor), an analog switch IC or the like may be used as the switch element (504).

The foregoing descriptions have been presented in order to explain the present invention by way of example, and a person having ordinary skill in the art which the present invention relates could make various modifications and variations. Accordingly, the foregoing embodiments disclosed shall be interpreted as being illustrative, while not being limitative, of the scope of the present invention. It should be understood that the scope of the present invention shall be defined by the Claims.

## Claims

1. A continuous glucose measurement apparatus, comprising:
a body attachable unit (20) configured to be insertedly attachable to a body to periodically measure blood glucose, the body attachable unit (20) comprising a housing (510), a battery (535), a PCB board (530) to be connected to the battery (535), and a sensor module (520) electrically connected to the PCB board (530) and arranged inside the housing (510) such that an end of the sensor module (520) outwardly protrudes from a bottom surface of the housing (510) being attachable to skin, and wherein the battery (535) and the PCB board (530) are mounted inside the housing (510);
an applicator (10) configured to outwardly discharge the body attachable unit (20) according to pressurizing manipulation of a press button; and
a protection cap (200) separatably coupled to the applicator (10) to block exposure of the body attachable unit (20) to an outside of the applicator (10) and configured to, in a state of being coupled to the applicator (10), prevent manipulation of pressing the press button;
wherein the body attachable unit (20) is arranged and coupled inside the applicator (10),
wherein the applicator (10) comprises a power supply switch means (PS1) configured to supply the power of the battery (535) to the body attachable unit (20),
**characterised in that** the power supply switch means (PS1) comprises:
a board contact point (501) formed at the PCB board (530);
a battery contact point (502) connected to the battery (535); and
a switch terminal (503) configured to contact both the board contact point (501) and the battery contact point (502) in a state when the protection cap (200) is removed from the applicator (10),
wherein the switch terminal (503) moves in association with the manipulation of the separation and removal of the protection cap (200) such that the switch terminal (503) contacts both the board contact point (501) and the battery contact point (502) simultaneously to electrically connect between the board contact point (501) and the battery contact point (502).

2. The continuous glucose measurement apparatus of claim 1, wherein the power supply switch means (PS1) comprises:
an elastic member (5031) configured to apply an elastic force to the switch terminal (503) in a direction in which the switch terminal (503) contacts the board contact point (501) and the battery contact point (502) simultaneous; and
a manipulation protrusion (205) formed to protrude at one side of the protection cap (200), the manipulation protrusion (205) configured to press the switch terminal (503) so that the switch terminal (503) is spaced apart from the board contact point (501) and the battery contact point (502).

3. The continuous glucose measurement apparatus of claim 2, wherein an insertion guide portion (513) protruded toward an inward direction so that insertion of the manipulation protrusion (205) of the protection cap (200) is guided is formed at the bottom surface of the housing (510).

4. The continuous glucose measurement apparatus of claim 3, wherein a blocking film (514) of a soft material configured to be deformable by pressure of the manipulation protrusion (205) and block an inner space of the housing (510) from an outside of the housing (510) is arranged at an end portion of protrusion of the insertion guide portion (513).

5. A continuous glucose measurement apparatus, comprising:
a body attachable unit (20) configured to be insertedly attachable to a body to periodically measure blood glucose, the body attachable unit (20) comprising a housing (510), a battery (535), a PCB board (530) to be connected to the battery (535), and a sensor module (520) electrically connected to the PCB board (530) and arranged inside the housing (510) such that an end of the sensor module (520) outwardly protrudes from a bottom surface of the housing (510) being attachable to skin, wherein the battery (535) and the PCB board (530) are mounted inside the housing (510);
an applicator (10) configured to outwardly discharge the body attachable unit (20) according to pressurizing manipulation of a press button; and
a protection cap (200) separatably coupled to the applicator (10) to block exposure of the body attachable unit (20) to an outside of the applicator (10) and configured to, in a state of being coupled to the applicator (10), prevent manipulation of pressing the press button;
wherein the body attachable unit (20) is arranged and coupled inside the applicator (10),
wherein the applicator (10) comprises a power supply switch means (PS2) configured to supply the power of the battery (535) to the body attachable unit (20) **characterised in that** the power supply switch means (PS2) is configured to supply the power in association with manipulation of separation and removal of the protection cap (200),
wherein the power supply switch means (PS2) comprises:
a switch element (504) configured to electrically connect or disconnect between the battery (535) and the PCB board (530) according to an electrical signal inputted to an enable terminal (5041);
a switch contact point (505) having one end grounded and an other end connected to the enable terminal (5041); and
a connection terminal (506) formed at one side of the protection cap (200) and configured to electrically connect or disconnect between the one end and the other end of the switch contact point (505) according to whether the protection cap (200) is separated and removed from the applicator (10) or not,
wherein a low electric signal is inputted to the enable terminal (5041) when the connection terminal (506) electrically connects between the one end and the other end of the switch contact point (505), and
wherein a high electric signal is inputted to the enable terminal (5041) by the power of the battery (535) when the connection terminal (506) electrically disconnects between the one end and the other end of the switch contact point (505).

6. The continuous glucose measurement apparatus of claim 5, wherein:
the switch contact point (505) is formed to be positioned in an inside space of the housing (510),
a manipulation protrusion (205) protruding toward the switch contact point (505) is formed at one side of the protection cap (200),
the connection terminal (506) is formed at an end portion of the manipulation protrusion (205), and
an insertion guide portion (513) protruding in an inward direction so that insertion of the manipulation protrusion (205) of the protection cap (200) is guided is formed at the bottom surface of the housing (510).

## Patentansprüche

1. Vorrichtung zur kontinuierlichen Glukosemessung, umfassend:
eine am Körper anbringbare Einheit (20), die dazu eingerichtet ist, durch Einführen an einem Körper anbringbar zu sein, um periodisch Blutglukose zu messen, wobei die am Körper anbringbare Einheit (20) ein Gehäuse (510), eine Batterie (535), eine mit der Batterie (535) zu verbindende Leiterplatte (530) und ein Sensormodul (520) umfasst, das elektrisch mit der Leiterplatte (530) verbunden und innerhalb des Gehäuses (510) derart angeordnet ist, dass ein Ende des Sensormoduls (520) von einer Bodenfläche des an der Haut anbringbaren Gehäuses (510) nach außen vorsteht, und wobei die Batterie (535) und die Leiterplatte (530) innerhalb des Gehäuses (510) montiert sind;
einen Applikator (10), der dazu eingerichtet ist, die am Körper anbringbare Einheit (20) entsprechend einer Druckbetätigung eines Druckknopfs nach außen auszugeben; und
eine Schutzkappe (200), die lösbar mit dem Applikator (10) gekoppelt ist, um zu verhindern, dass die am Körper anbringbare Einheit (20) zur Außenseite des Applikators (10) hin freiliegt, und die dazu eingerichtet ist, in einem mit dem Applikator (10) gekoppelten Zustand eine Betätigung durch Drücken des Druckknopfs zu verhindern;
wobei die am Körper anbringbare Einheit (20) innerhalb des Applikators (10) angeordnet und gekoppelt ist,
wobei der Applikator (10) ein Stromversorgungsschaltmittel (PS1) umfasst, das dazu eingerichtet ist, die Leistung der Batterie (535) der am Körper anbringbaren Einheit (20) zuzuführen,
**dadurch gekennzeichnet, dass** das Stromversorgungsschaltmittel (PS1) umfasst:
einen an der Leiterplatte (530) ausgebildeten Leiterplattenkontaktpunkt (501);
einen mit der Batterie (535) verbundenen Batteriekontaktpunkt (502); und
einen Schaltanschluss (503), der dazu eingerichtet ist, in einem Zustand, in dem die Schutzkappe (200) vom Applikator (10) entfernt ist, sowohl den Leiterplattenkontaktpunkt (501) als auch den Batteriekontaktpunkt (502) zu kontaktieren,
wobei sich der Schaltanschluss (503) im Zusammenhang mit der Betätigung zum Trennen und Entfernen der Schutzkappe (200) derart bewegt, dass der Schaltanschluss (503) sowohl den Leiterplattenkontaktpunkt (501) als auch den Batteriekontaktpunkt (502) gleichzeitig kontaktiert, um den Leiterplattenkontaktpunkt (501) und den Batteriekontaktpunkt (502) elektrisch miteinander zu verbinden.

2. Vorrichtung zur kontinuierlichen Glukosemessung nach Anspruch 1, wobei das Stromversorgungsschaltmittel (PS1) umfasst:
ein elastisches Element (5031), das dazu eingerichtet ist, eine elastische Kraft auf den Schaltanschluss (503) in einer Richtung auszuüben, in der der Schaltanschluss (503) den Leiterplattenkontaktpunkt (501) und den Batteriekontaktpunkt (502) gleichzeitig kontaktiert; und
einen Betätigungsvorsprung (205), der so ausgebildet ist, dass er an einer Seite der Schutzkappe (200) vorsteht, wobei der Betätigungsvorsprung (205) dazu eingerichtet ist, auf den Schaltanschluss (503) zu drücken, sodass der Schaltanschluss (503) von dem Leiterplattenkontaktpunkt (501) und dem Batteriekontaktpunkt (502) beabstandet ist.

3. Vorrichtung zur kontinuierlichen Glukosemessung nach Anspruch 2, wobei an der Bodenfläche des Gehäuses (510) ein Einführführungsabschnitt (513) ausgebildet ist, der in einer nach innen gerichteten Richtung vorsteht, sodass das Einführen des Betätigungsvorsprungs (205) der Schutzkappe (200) geführt wird.

4. Vorrichtung zur kontinuierlichen Glukosemessung nach Anspruch 3, wobei eine Sperrfolie (514) aus einem weichen Material, die dazu eingerichtet ist, durch den Druck des Betätigungsvorsprungs (205) verformbar zu sein und einen Innenraum des Gehäuses (510) gegenüber einer Außenseite des Gehäuses (510) abzuschirmen, an einem Endabschnitt des Vorsprungs des Einführführungsabschnitts (513) angeordnet ist.

5. Vorrichtung zur kontinuierlichen Glukosemessung, umfassend:
eine am Körper anbringbare Einheit (20), die dazu eingerichtet ist, durch Einführen an einem Körper anbringbar zu sein, um periodisch Blutglukose zu messen, wobei die am Körper anbringbare Einheit (20) ein Gehäuse (510), eine Batterie (535), eine mit der Batterie (535) zu verbindende Leiterplatte (530) und ein Sensormodul (520) umfasst, das elektrisch mit der Leiterplatte (530) verbunden und innerhalb des Gehäuses (510) derart angeordnet ist, dass ein Ende des Sensormoduls (520) von einer Bodenfläche des an der Haut anbringbaren Gehäuses (510) nach außen vorsteht, wobei die Batterie (535) und die Leiterplatte (530) innerhalb des Gehäuses (510) montiert sind;
einen Applikator (10), der dazu eingerichtet ist, die am Körper anbringbare Einheit (20) entsprechend einer Druckbetätigung eines Druckknopfs nach außen auszugeben; und
eine Schutzkappe (200), die lösbar mit dem Applikator (10) gekoppelt ist, um zu verhindern, dass die am Körper anbringbare Einheit (20) zur Außenseite des Applikators (10) hin freiliegt, und die dazu eingerichtet ist, in einem mit dem Applikator (10) gekoppelten Zustand eine Betätigung durch Drücken des Druckknopfs zu verhindern;
wobei die am Körper anbringbare Einheit (20) innerhalb des Applikators (10) angeordnet und gekoppelt ist,
wobei der Applikator (10) ein Stromversorgungsschaltmittel (PS2) umfasst, das dazu eingerichtet ist, die Leistung der Batterie (535) der am Körper anbringbaren Einheit (20) zuzuführen,
**dadurch gekennzeichnet, dass** das Stromversorgungsschaltmittel (PS2) dazu eingerichtet ist, die Leistung im Zusammenhang mit einer Betätigung zum Trennen und Entfernen der Schutzkappe (200) zuzuführen,
wobei das Stromversorgungsschaltmittel (PS2) umfasst:
ein Schaltelement (504), das dazu eingerichtet ist, die Batterie (535) und die Leiterplatte (530) entsprechend einem an einen Freigabeanschluss (5041) angelegten elektrischen Signal elektrisch miteinander zu verbinden oder voneinander zu trennen;
einen Schaltkontaktpunkt (505), der ein mit Masse verbundenes Ende und ein anderes, mit dem Freigabeanschluss (5041) verbundenes Ende aufweist; und
einen Verbindungsanschluss (506), der an einer Seite der Schutzkappe (200) ausgebildet und dazu eingerichtet ist, das eine Ende und das andere Ende des Schaltkontaktpunkts (505) elektrisch miteinander zu verbinden oder voneinander zu trennen, je nachdem, ob die Schutzkappe (200) vom Applikator (10) getrennt und entfernt ist oder nicht,
wobei ein elektrisches Signal mit niedrigem Pegel an den Freigabeanschluss (5041) angelegt wird, wenn der Verbindungsanschluss (506) das eine Ende und das andere Ende des Schaltkontaktpunkts (505) elektrisch miteinander verbindet, und
wobei durch die Leistung der Batterie (535) ein elektrisches Signal mit hohem Pegel an den Freigabeanschluss (5041) angelegt wird, wenn der Verbindungsanschluss (506) das eine Ende und das andere Ende des Schaltkontaktpunkts (505) elektrisch voneinander trennt.

6. Vorrichtung zur kontinuierlichen Glukosemessung nach Anspruch 5, wobei:
der Schaltkontaktpunkt (505) so ausgebildet ist, dass er in einem Innenraum des Gehäuses (510) positioniert ist,
an einer Seite der Schutzkappe (200) ein Betätigungsvorsprung (205) ausgebildet ist, der in Richtung des Schaltkontaktpunkts (505) vorsteht,
der Verbindungsanschluss (506) an einem Endabschnitt des Betätigungsvorsprungs (205) ausgebildet ist, und
an der Bodenfläche des Gehäuses (510) ein Einführführungsabschnitt (513) ausgebildet ist, der in einer nach innen gerichteten Richtung vorsteht, sodass das Einführen des Betätigungsvorsprungs (205) der Schutzkappe (200) geführt wird.

## Revendications

1. Dispositif de mesure continue de glycémie, comprenant :
une unité fixable sur le corps (20), prévue pour être fixée par insertion sur un corps afin de mesurer périodiquement la glycémie, ladite unité fixable sur un corps (20) comprenant un boîtier (510), une batterie (535), une carte de circuit imprimé (530) à connecter à la batterie (535), et un module de capteur (520) connecté électriquement à la carte de circuit imprimé (530) et disposé à l'intérieur du boîtier (510) de sorte qu'une extrémité du module de capteur (520) fait saillie vers l'extérieur depuis une surface inférieure du boîtier (510) fixable sur la peau, et où la batterie (535) la carte de circuit imprimé (530) sont montées à l'intérieur du boîtier (510) ;
un applicateur (10) prévu pour décharger vers l'extérieur l'unité fixable sur le corps (20) en fonction de la manipulation par pression d'un bouton-poussoir ; et
un capuchon de protection (200) raccordé de manière séparable à l'applicateur (10) pour empêcher l'exposition de l'unité fixable sur le corps (20) à l'extérieur de l'applicateur (10) et prévu pour empêcher la manipulation par pression du bouton-poussoir, dans un état où il est raccordé à l'applicateur (10) ;
où l'unité fixable sur le corps (20) est disposée et raccordée à l'intérieur de l'applicateur (10), où l'applicateur (10) comprend un moyen de commutation d'alimentation électrique (PS1) prévu pour fournir la puissance de la batterie (535) à l'unité fixable sur le corps (20), **caractérisé en ce que**
le moyen de commutation d'alimentation (PS1) comprend :
un point de contact de carte (501) formé sur la carte de circuit imprimé (530) ;
un point de contact de batterie (502) connecté à la batterie (535) ; et
une borne de commutation (503) prévue pour entrer en contact à la fois avec le point de contact de carte (501) et le point de contact de batterie (502) dans un état où le capuchon de protection (200) est retiré de l'applicateur (10),
où la borne de commutation (503) se déplace en lien avec la manipulation de la séparation et le retrait du capuchon de protection (200), de sorte que la borne de commutation (503) entre en contact à la fois avec le point de contact de carte (501) et le point de contact de batterie (502) pour une connexion électrique simultanée entre le point de contact de carte (501) et le point de contact de batterie (502).

2. Dispositif de mesure continue de glycémie selon la revendication 1, où le moyen de commutation d'alimentation (PS1) comprend :
un élément élastique (5031) prévu pour appliquer une force élastique à la borne de commutation (503) dans une direction où la borne de commutation (503) entre en contact simultanément avec le point de contact de carte (501) et le point de contact de batterie (502) ; et
une saillie de manipulation (205) formée de manière à faire saillie d'un côté du capuchon de protection (200), ladite saillie de manipulation (205) étant prévue pour exercer une pression sur la borne de commutation (503) de manière à espacer la borne de commutation (503) du point de contact de carte (501) et du point de contact de batterie (502).

3. Dispositif de mesure continue de glycémie selon la revendication 2, où une partie de guidage d'insertion (513) faisant saillie vers l'intérieur de manière à guider l'insertion de la saillie de manipulation (205) du capuchon de protection (200) est formée sur la surface inférieure du boîtier (510).

4. Dispositif de mesure continue de glycémie selon la revendication 3, où un film de blocage (514) dans une matière souple prévue pour être déformable par pression de la saillie de manipulation (205) et bloquer un espace intérieur du boîtier (510) depuis l'extérieur du boîtier (510) est disposée dans une partie d'extrémité de la saillie de la partie de guidage d'insertion (513).

5. Dispositif de mesure continue de glycémie, comprenant :
une unité fixable sur le corps (20), prévue pour être fixée par insertion sur un corps afin de mesurer périodiquement la glycémie, ladite unité fixable sur un corps (20) comprenant un boîtier (510), une batterie (535), une carte de circuit imprimé (530) à connecter à la batterie (535), et un module de capteur (520) connecté électriquement à la carte de circuit imprimé (530) et disposé à l'intérieur du boîtier (510) de sorte qu'une extrémité du module de capteur (520) fait saillie vers l'extérieur depuis une surface inférieure du boîtier (510) fixable sur la peau, et où la batterie (535) la carte de circuit imprimé (530) sont montées à l'intérieur du boîtier (510) ;
un applicateur (10) prévu pour décharger vers l'extérieur l'unité fixable sur le corps (20) en fonction de la manipulation par pression d'un bouton-poussoir ; et
un capuchon de protection (200) raccordé de manière séparable à l'applicateur (10) pour empêcher l'exposition de l'unité fixable sur le corps (20) à l'extérieur de l'applicateur (10) et prévu pour empêcher la manipulation par pression du bouton-poussoir, dans un état où il est raccordé à l'applicateur (10) ;
où l'unité fixable sur le corps (20) est disposée et raccordée à l'intérieur de l'applicateur (10), où l'applicateur (10) comprend un moyen de commutation d'alimentation électrique (PS1) prévu pour fournir la puissance de la batterie (535) à l'unité fixable sur le corps (20), **caractérisé en ce que**
le moyen de commutation d'alimentation électrique (PS2) est prévu pour fournir l'énergie en lien avec la manipulation de la séparation et du retrait du capuchon de protection (200), le moyen de commutation d'alimentation électrique (PS2) comprenant :
un élément de commutation (504) prévu pour connecter ou déconnecter électriquement la batterie (535) et la carte de circuit imprimé (530) en fonction d'un signal électrique appliqué à une borne d'activation (5041) ;
un point de contact de commutation (505) ayant une extrémité mise à la terre et une autre extrémité connectée à la borne d'activation (5041) ; et
une borne de connexion (506) formée d'un côté du capuchon de protection (200) et prévue pour connecter ou déconnecter électriquement la première extrémité et l'autre extrémité du point de contact de commutation (505) selon que le capuchon de protection (200) est séparé et retiré de l'applicateur (10) ou pas,
où un signal électrique faible est appliqué à la borne d'activation (5041) lorsque la borne de connexion (506) connecte électriquement une extrémité et l'autre extrémité du point de contact de commutation (505), et
où un signal électrique élevé est appliqué à la borne d'activation (5041) par l'alimentation de la batterie (535) lorsque la borne de connexion (506) déconnecte électriquement une extrémité et l'autre extrémité du point de contact de commutation (505).

6. Dispositif de mesure continue de glycémie selon la revendication 5, où :
le point de contact de commutation (505) est formé de manière à être positionné dans un espace intérieur du boîtier (510),
une saillie de manipulation (205) faisant saillie vers le point de contact de commutation (505) est formée d'un côté du capuchon de protection (200),
la borne de connexion (506) est formée dans une partie d'extrémité de la saillie de manipulation (205), et
une partie de guidage d'insertion (513) faisant saillie vers l'intérieur de manière à guider l'insertion de la saillie de manipulation (205) du capuchon de protection (200) est formée sur la surface inférieure du boîtier (510).
